# EUROPEAN PATENT APPLICATION

(11) **EP 3 845 634 A1**
(43) Date of publication of application: **07.07.2021**
(21) Application number: 19854638.4
(22) Date of filing: 30.08.2019
(51) Int. Cl.: C12N 5/02, A61K 9/19, A61K 35/30, A61K 47/02, A61K 47/20, A61K 47/26, A61K 47/42, A61L 27/38, A61P 27/02, A61P 43/00, C12N 1/04, C12N 5/077, C07K 14/76

(54) **COMPOSITION AND METHOD FOR PRESERVING OR CULTURING OCULAR CELLS**

(30) Priority: 31.08.2018 JP 2018163238
(71) Applicant: The Doshisha, Kyoto-shi, Kyoto 602-8580 (JP)
(72) Inventor: KOIZUMI Noriko, Kyotanabe-shi, Kyoto 610-0394 (JP); OKUMURA Naoki, Kyotanabe-shi, Kyoto 610-0394 (JP)
(74) Representative: J A Kemp LLP
(86) International application number: PCT/JP2019/034176
(87) International publication number: WO 2020/045642

(57) **Abstract**

The present disclosure provides a composition and method for preserving ocular cells. More specifically, the present disclosure provides a composition for preserving ocular cells, or culturing the cells after preservation, comprising albumin and dimethyl sulfoxide, and a cell formulation comprising the ocular cells, albumin, and dimethyl sulfoxide, and a treatment/prevention method using the same. The present disclosure also provides a method of preserving corneal endothelial cells, comprising suspending ocular cells in the composition to provide a suspension and freezing the suspension. In some embodiments, ocular cells are corneal cells (e.g., corneal endothelial cells).

## Description

### [Technical Field]

The present disclosure relates to a technology for preserving ocular cells, or culturing the cells after preservation.

### [Background Art]

Visual information is recognized when light transmitted into the cornea, which is a transparent tissue at the front-most part of an eye ball, reaches the retina and excites nerve cells of the retina, and a generated electric signal is transmitted through the optic nerve to the visual cortex of the cerebrum. To attain good vision, it is necessary that the cornea is transparent. The transparency of the cornea is maintained by maintaining constant water content by the pump and barrier functions of corneal endothelial cells.

While human corneal endothelial cells are present at a density of about 3000 cell per 1 mm² at birth, once severely damaged, corneal endothelial cells cannot maintain their function such that transparency of the cornea is lost due to the limited ability of the cells to regenerate. Corneal endothelial dystrophy or bullous keratopathy induced by corneal endothelial dysfunction due to various causes results in edema or turbidity of the cornea, leading to significant deterioration in vision. Currently, heterologous corneal transplant using a donor cornea is performed for bullous keratopathy. However, many problems remain to be solved for corneal transplantation, such as invasiveness of surgery, rejection, and shortage in donors.

To overcome such problems, cell transplant therapy with low invasiveness have been developed in recent years as therapy for corneal endothelial diseases (Non Patent Literature 1). Culture of corneal endothelial cells is challenging, and involves problems such as inability to grow with conventional methods (Non Patent Literatures 2 and 3), fibroblast formation (Non Patent Literature 4), and cellular senescence (Non Patent Literature 5). Culturing methods of corneal endothelial cells are being actively studied, and in fact have become clinically applicable (Non Patent Literature 1).

### [Citation List]

### [Non Patent Literature]

[NPL 1] Kinoshita S, Koizumi N, Ueno M, et al. Injection of cultured cells with a ROCK inhibitor for bullous keratopathy. N Engl J Med 2018; 378: 995-1003.
[NPL 2] Okumura N, Ueno M, Koizumi N, et al. Enhancement on primate corneal endothelial cell survival in vitro by a ROCK inhibitor. Invest Ophthalmol Vis Sci 2009; 50: 3680-3687.
[NPL 3] Nakahara M, Okumura N, Kay EP, et al. Corneal endothelial expansion promoted by human bone marrow mesenchymal stem cell-derived conditioned medium. PLoS One 2013; 8: e69009.
[NPL 4] Okumura N, Kay EP, Nakahara M, Hamuro J, Kinoshita S, Koizumi N. Inhibition of TGF-beta Signaling Enables Human Corneal Endothelial Cell Expansion In Vitro for Use in Regenerative Medicine. PLoS One 2013; 8: e58000.
[NPL 5] Hongo A, Okumura N, Nakahara M, Kay EP, Koizumi N. The Effect of a p38 Mitogen-Activated Protein Kinase Inhibitor on Cellular Senescence of Cultivated Human Corneal Endothelial Cells. Invest Ophthalmol Vis Sci 2017; 58: 3325-3334.

### [Summary of Invention]

### [Solution to Problem]

The inventors have come to discover a method of stably preserving ocular cells (e.g., corneal cells, typically corneal endothelial cells) that can withstand long-term preservation (e.g., cryopreservation) and maintain a cell survival rate and/or cell density after preservation as a result of diligent study. The inventors have also come to apply such a preservation method to provide a cell formulation that can be used directly (ready-to-use) in ocular cell transplant and a therapeutic or prophylactic method using the same. The present disclosure also provides a method of preserving cells that can provide a cell formulation, which can be directly used substantially without additional manipulation, and a composition and method for preservation that can be used in said method.

Specifically, the inventors found that the cell survival rate and/or cell density is maintained and the cell count in culture after preservation is significantly increased by preserving ocular cells (e.g., corneal cells, typically corneal endothelial cells) by using a specific component, especially a component that can be used in clinical applications after preservation. Surprisingly, the cell survival rate and cell density were higher with preservation using a specific medium used herein as compared to using a preservation medium based on Opti-MEM®-I, which is conventionally considered to be the most suitable for culture of corneal endothelial cells, with an addition of DMSO and FBS. The inventors also surprisingly found that ocular cells preserved by such a preservation method can be used in clinical settings even when the cells are not subsequently cultured.

Therefore, the present disclosure provides the following.
(Item 1) A composition for use in preserving ocular cells, or culturing the cells after preservation, comprising albumin and dimethyl sulfoxide.
(Item 2) The composition of item 1, wherein the ocular cells comprise corneal cells.
(Item 3) The composition of any one of the preceding items, wherein the ocular cells comprise corneal endothelial cells.
(Item 4) The composition of any one of the preceding items, wherein the albumin is derived from a cow.
(Item 5) The composition of any one of the preceding items, further comprising a saccharide.
(Item 6) The composition of any one of the preceding items, wherein the saccharide is a monosaccharide.
(Item 7) The composition of any one of the preceding items, wherein the saccharide is a glucose.
(Item 8) The composition of any one of the preceding items, further comprising a phosphate ion.
(Item 9) The composition of any one of the preceding items, further comprising a metal ion.
(Item 10) The composition of any one of the preceding items, wherein the metal ion is a divalent metal ion.
(Item 11) The composition of any one of the preceding items, wherein the metal ion is selected from the group consisting of a calcium ion, a magnesium ion, an iron ion, a zinc ion, a copper ion, an aluminum ion, and any combination thereof.
(Item 12) The composition of any one of the preceding items, wherein the composition comprises (i) a calcium ion and magnesium or (ii) calcium, magnesium, and copper.
(Item 13) The composition of any one of the preceding items, wherein a concentration of the purified albumin is within the range of 0.01 to 25% (w/w).
(Item 14) The composition of any one of the preceding items, wherein a concentration of the purified albumin is within the range of 0.1 to 5% (w/w).
(Item 15) The composition of any one of the preceding items, wherein a concentration of the dimethyl sulfoxide is within the range of 0.1 to 50% (w/w).
(Item 16) The composition of any one of the preceding items, wherein a concentration of the dimethyl sulfoxide is within the range of 1 to 20% (w/w).
(Item 17) The composition of any one of the preceding items, wherein a concentration of the metal ion is within the range of 0.0000001 to 5% (w/w).
(Item 18) The composition of any one of the preceding items, wherein a concentration of the metal ion is within the range of 0.1 to 5% (w/w).
(Item 19) The composition of any one of the preceding items, wherein the composition comprises (i) 0.1% calcium and magnesium or (ii) 0.1 to 5% calcium, magnesium, and copper.
(Item 20) The composition of any one of the preceding items, wherein a concentration of the saccharide is within the range of 0.1 to 10% (w/w).
(Item 21) The composition of any one of the preceding items, wherein a concentration of the saccharide is within the range of 1 to 5% (w/w).
(Item 22) The composition of any one of the preceding items, wherein the preservation is cryopreservation.
(Item 23) The composition of any one of the preceding items, wherein the composition is Bambanker®.
(Item 24) A method for preserving corneal endothelial cells, comprising:
   suspending ocular cells in the composition of any one of the preceding items to provide a suspension; and
   freezing the suspension.
(Item 25) The method of any one of the preceding items, wherein the corneal endothelial cells are characterized by being suspended in the composition at 1 × 10³ to 1 × 10⁸ cells/ml.
(Item 26) The method of any one of the preceding items, wherein freezing the suspension comprises freezing without pre-freezing.
(Item 27) The method of any one of the preceding items, wherein the freezing without pre-freezing comprises freezing rapidly at -80°C.
(Item 28) A frozen cell product comprising corneal endothelial cells that have been cryopreserved in accordance with the method of any one of the preceding items.
(Item 1A) A composition for use in preserving ocular cells, or culturing the cells after preservation, comprising albumin and dimethyl sulfoxide.
(Item 2A) The composition of the preceding item, wherein the ocular cells comprise corneal cells.
(Item 3A) The composition of any one of the preceding items, wherein the ocular cells comprise corneal endothelial cells.
(Item 4A) The composition of any one of the preceding items, wherein the ocular cells are isolated cells or cells derived from progenitor cells.
(Item 5A) The composition of any one of the preceding items, wherein the albumin is derived from a cow.
(Item 6A) The composition of any one of the preceding items, further comprising a saccharide.
(Item 7A) The composition of any one of the preceding items, wherein the saccharide is a monosaccharide.
(Item 8A) The composition of any one of the preceding items, wherein the saccharide is a glucose.
(Item 9A) The composition of any one of the preceding items, further comprising a phosphate ion.
(Item 10A) The composition of any one of the preceding items, further comprising a metal ion.
(Item 11A) The composition of any one of the preceding items, wherein the metal ion is a divalent metal ion.
(Item 12A) The composition of any one of the preceding items, wherein the metal ion is selected from the group consisting of a calcium ion, a magnesium ion, an iron ion, a zinc ion, a copper ion, an aluminum ion, and any combination thereof.
(Item 13A) The composition of item 10A, wherein the composition comprises (i) a calcium ion and magnesium or (ii) calcium, magnesium, and copper.
(Item 14A) The composition of any one of the preceding items, wherein a concentration of the albumin is within the range of about 0.01 to about 25% (w/w).
(Item 15A) The composition of any one of the preceding items, wherein a concentration of the albumin is within the range of about 0.1 to about 5% (w/w).
(Item 16A) The composition of any one of the preceding items, wherein a concentration of the albumin is about 1% (w/w).
(Item 17A) The composition of any one of the preceding items, wherein a concentration of the dimethyl sulfoxide is within the range of about 0.1 to about 50% (w/w).
(Item 18A) The composition of any one of the preceding items, wherein a concentration of the dimethyl sulfoxide is within the range of about 1 to about 20% (w/w).
(Item 19A) The composition of any one of the preceding items, wherein a concentration of the dimethyl sulfoxide is about 10% (w/w).
(Item 20A) The composition of any one of the preceding items, wherein a concentration of the metal ion is within the range of about 0.0000001 to 5% (w/w).
(Item 21A) The composition of any one of items 10A to 19A, wherein a concentration of the metal ion is within the range of about 0.1 to about 5% (w/w).
(Item 22A) The composition of any one of the preceding items, wherein the composition comprises (i) about 0.1% calcium and magnesium or (ii) about 0.1 to about 5% calcium, magnesium, and copper.
(Item 23A) The composition of any one of the preceding items, wherein a concentration of the saccharide is within the range of about 0.1 to about 10% (w/w).
(Item 24A) The composition of any one of the preceding items, wherein a concentration of the saccharide is within the range of about 1 to about 5% (w/w).
(Item 25A) The composition of any one of the preceding items, wherein the preservation is cryopreservation.
(Item 26A) The composition of any one of the preceding items, wherein the composition is Bambanker®.
(Item 27A) The composition of any one of the preceding items, wherein the composition is for administering corneal endothelial cells after preservation to a subject.
(Item 28A) The composition of any one of the preceding items, wherein the corneal endothelial cells after preservation are characterized by being administered to a subject without culturing.
(Item 29A) The composition of any one of the preceding items, wherein the corneal endothelial cells are characterized by being co-administered with a ROCK inhibitor.
(Item 30A) The composition of any one of the preceding items, wherein the ROCK inhibitor is selected from Y-27632 ((+)-trans-4-(1-aminoethyl)-1-(4-pyridylcarbamoyl)cyclohex a ne), ripasudil (4-fluoro-5-{[(2S)-2-methyl-1,4-diazepan-1-yl]sulfonyl}isoq uinoline), fasudil (1-(5-isoquinolinesulfonyl)homopiperadine), or a pharmaceutically acceptable salt thereof.
(Item 31A) A method for preserving ocular cells, comprising:
   suspending ocular cells in the composition of any one of the preceding items to provide a suspension; and
   freezing the suspension.
(Item 32A) The method of any one of the preceding items, wherein the ocular cells are characterized by being suspended in the composition at about 1 × 10³ to about 1 × 10⁸ cells/ml.
(Item 33A) The method of any one of the preceding items, wherein the freezing the suspension comprises freezing without pre-freezing.
(Item 34A) The method of any one of the preceding items, wherein the freezing without pre-freezing comprises freezing rapidly at about -80°C.
(Item 35A) A frozen cell product comprising corneal endothelial cells that have been cryopreserved in accordance with the method of any one of the preceding items.
(Item 36A) A cell formulation comprising corneal endothelial cells, albumin, and dimethyl sulfoxide.
(Item 37A) The cell formulation of any one of the preceding items, provided in a frozen state.
(Item 38A) The cell formulation of any one of the preceding items for treating or preventing a corneal endothelial disease, disorder, or symptom.
(Item 39A) The cell formulation of any one of the preceding items, wherein the albumin is derived from a cow.
(Item 40A) The cell formulation of any one of the preceding items, further comprising a saccharide.
(Item 41A) The cell formulation of any one of the preceding items, wherein the saccharide is a monosaccharide.
(Item 42A) The cell formulation of any one of the preceding items, wherein the saccharide is a glucose.
(Item 43A) The cell formulation of any one of the preceding items, further comprising a phosphate ion.
(Item 44A) The cell formulation of any one of the preceding items, further comprising a metal ion.
(Item 45A) The cell formulation of any one of the preceding items, wherein the metal ion is a divalent metal ion.
(Item 46A) The cell formulation of any one of the preceding items, wherein the metal ion is selected from the group consisting of a calcium ion, a magnesium ion, an iron ion, a zinc ion, a copper ion, an aluminum ion, and any combination thereof.
(Item 47A) The cell formulation of any one of the preceding items, wherein the cell formulation comprises (i) a calcium ion and a magnesium ion or (ii) a calcium ion, a magnesium ion, and a copper ion.
(Item 48A) The cell formulation of any one of the preceding items, wherein a concentration of the albumin is within the range of about 0.01 to about 25% (w/w).
(Item 49A) The cell formulation of any one of the preceding items, wherein a concentration of the albumin is within the range of about 0.1 to about 5% (w/w).
(Item 50A) The cell formulation of any one of the preceding items, wherein a concentration of the dimethyl sulfoxide is within the range of about 0.1 to about 50% (w/w).
(Item 51A) The cell formulation of any one of the preceding items, wherein a concentration of the dimethyl sulfoxide is within the range of about 1 to about 20% (w/w).
(Item 52A) The cell formulation of any one of the preceding items, wherein a concentration of the metal ion is within the range of about 0.0000001 to about 5% (w/w).
(Item 53A) The cell formulation of any one of the preceding items, wherein a concentration of the metal ion is within the range of about 0.1 to about 5% (w/w).
(Item 54A) The cell formulation of any one of the preceding items, wherein the cell formulation comprises (i) about 0.1% calcium ion and magnesium ion or (ii) about 0.1 to about 5% calcium ion, magnesium ion, and copper ion.
(Item 55A) The cell formulation of any one of the preceding items, wherein a concentration of the saccharide is within the range of about 0.1 to about 10% (w/w).
(Item 56A) The cell formulation of any one of the preceding items, wherein a concentration of the saccharide is within the range of about 1 to about 5% (w/w).
(Item 57A) The cell formulation of any one of the preceding items, wherein the corneal endothelial cells are at a concentration of about 1 × 10³ to about 1 × 10⁸ cells/ml.
(Item 58A) The cell formulation of any one of the preceding items, further comprising a ROCK inhibitor.
(Item 59A) The cell formulation of any one of the preceding items, wherein the ROCK inhibitor is selected from Y-27632, ripasudil, fasudil, or a pharmaceutically acceptable salt thereof.
(Item 60A) A method for treating or preventing a corneal endothelial disease, disorder, or symptom in a subject, comprising administering to the subject a therapeutically effective amount of corneal endothelial cells preserved in accordance with the method of any one of items 31A to 34A.
(Item 61A) The method of any one of the preceding items, wherein the corneal endothelial cells are administered in conjunction with a ROCK inhibitor.
(Item 62A) The method of any one of the preceding items, wherein the ROCK inhibitor is selected from Y-27632, ripasudil, fasudil, or a pharmaceutically acceptable salt thereof.
(Item 63A) The method of any one of the preceding items without a step of culturing corneal endothelial cells.
(Item 64A) Corneal endothelial cells for treating or preventing a corneal endothelial disease, disorder, or symptom in a subject, wherein the corneal endothelial cells are corneal endothelial cells preserved in accordance with the method of any one of the preceding items.
(Item 65A) The corneal endothelial cells of any one of the preceding items, wherein the corneal endothelial cells are characterized by being administered in conjunction with a ROCK inhibitor.
(Item 66A) The corneal endothelial cells of any one of the preceding items, wherein the ROCK inhibitor is selected from Y-27632, ripasudil, fasudil, or a pharmaceutically acceptable salt thereof.
(Item 67A) The method of any one of the preceding items, wherein corneal endothelial cells after preservation are characterized by being administered without further culturing.
   Use of corneal endothelial cells in the manufacture of a drug for treating or preventing corneal endothelial disease, disorder, or symptom in a subject, wherein the corneal endothelial cells are corneal endothelial cells preserved in accordance with the method of any one of items 31A to 34A.
(Item 69A) The use of any one of the preceding items, wherein the corneal endothelial cells are characterized by being administered in conjunction with a ROCK inhibitor.
(Item 70A) The use of any one of the preceding items, wherein the ROCK inhibitor is selected from Y-27632, ripasudil, fasudil, or a pharmaceutically acceptable salt thereof.
(Item 71A) The use of any one of the preceding items, wherein corneal endothelial cells after preservation are characterized by being administered without further culturing.

The present disclosure is intended so that one or more of the aforementioned features can be provided not only as the explicitly disclosed combinations, but also as other combinations thereof. Additional embodiments and advantages of the present disclosure are recognized by those skilled in the art by reading and understanding the following detailed description as needed.

### [Advantageous Effects of Invention]

According to the present disclosure, ocular cells (e.g., corneal cells, typically corneal endothelial cells) can be preserved at a high cell survival rate and cell density. The cell count also significantly increases by culturing after preservation. Preserved cells can be used in clinical settings even when the cells are not subsequently cultured.

In this present disclosure, a formulation comprising ocular cells (e.g., corneal cells, typically corneal endothelial cells) can be clinically used directly after preservation. Thus, a so-called ready-to-use cell formulation is provided. In regenerative medicine, cumbersome operations are preferably omitted as much as possible in clinical settings . Thus, the present disclosure drastically improves the work efficiency in transplant surgery settings.

### [Brief Description of Drawings]

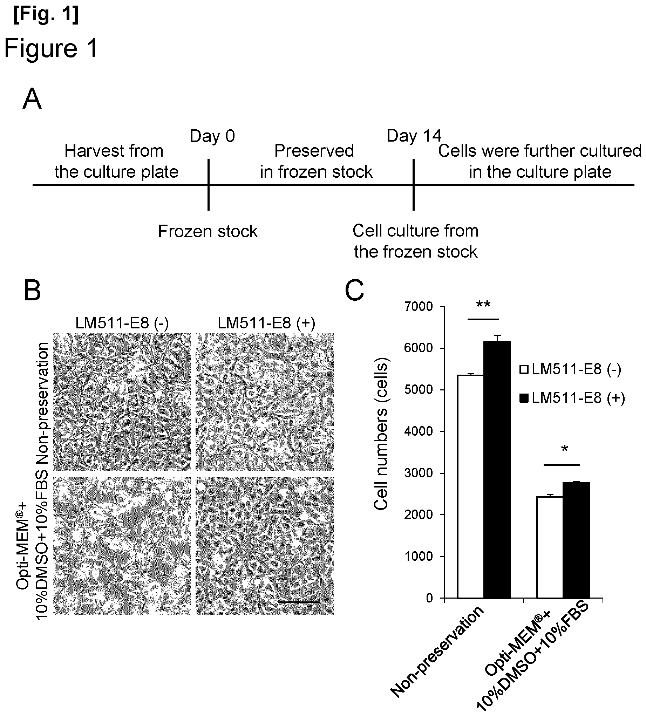

[Figure 1] Figure 1 shows experimental results from cryopreservation of human corneal endothelial cells (HCECs) using Opti-MEM® + 10% DMSO + 10% FBS. (A) Figure 1 shows a chronological schedule for HCEC cryopreservation. HCECs were harvested from a culture plate and suspended in a cryopreservation reagent. After 14 days of cryopreservation, the HCECs were seeded on a culture plate and cultured. (B) HCECs were suspended in Opti-MEM® + 10% DMSO + 10% FBS, cryopreserved for 14 days, initiated, seeded, and cultured for 24 hours. Phase contrast microscope pictures show that fewer cells are attached to a culture plate due to cell death of HCECs during preservation, compared to HCECs (top left) cultured with no prior cryopreservation shown as the control (bottom left) . The number of surviving HCECs increased by coating a culture dish with laminin 511 E8 fragments. Scale bar: 200 µm. (C) The cell count of unpreserved HCECs and cryopreserved HCECs at 24 hours after culture significantly increased by laminin 511 E8 fragment coating after 24 hours of culture. However, the cryopreserved HCEC count was about half of the cell count obtained for the unpreserved HCECs. The experiments were conducted three times each (n = 3). ** p < 0.01, * p < 0.05.

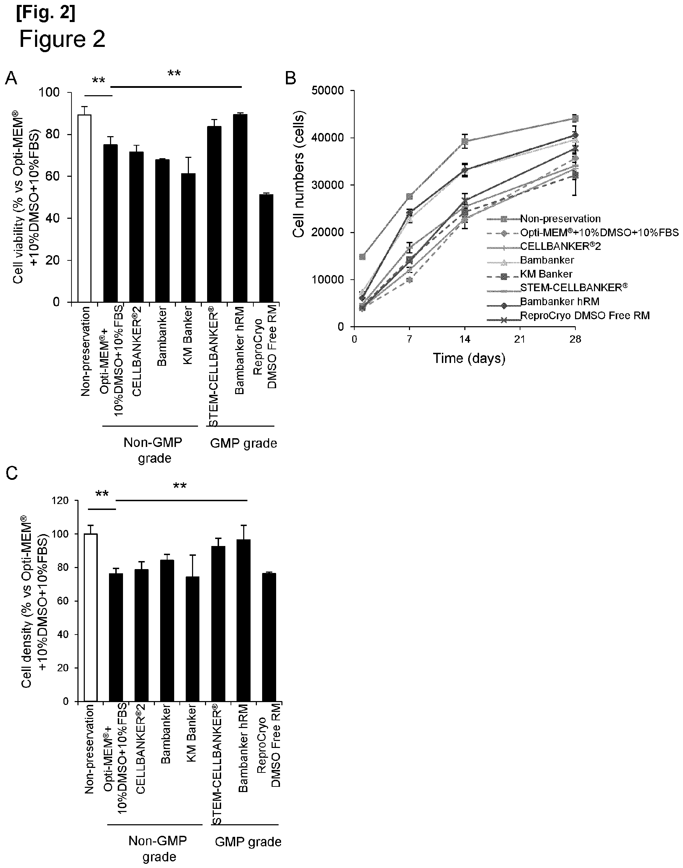

[Figure 2] Figure 2 shows screening of preservation reagents for cryopreservation. (A) Human corneal endothelial cells (HCECs) were cryopreserved for 14 days using various reagents. HCECs were resuspended in a medium, and the cell survival rate was calculated by trypan blue staining. The survival rate of the HCECs was about 75% when Opti-MEM® + 10% DMSO + 10% FBS was used. Use of Bambanker hRM as a preservation reagent significantly increased the cell survival rate to 89.4%. The experiments were conducted three times each. Statistical significance (vs. Opti-MEM® + 10% DMSO + 10% FBS) was calculated by Dunnett's multiple comparison test (n = 3). ** p < 0.01. (B) HCECs were cultured for 28 days after cryopreservation. The preserved HCECs all grew over 28 days, but cells that were cryopreserved in Bambanker hRM and Bambanker tended to grow more rapidly than HCECs preserved in another medium. The experiments were repeated (n = 3). (C) The cell density after cryopreservation and 28 days of culture was evaluated with ImageJ. The cell density after cryopreservation in Opti-MEM® + 10% DMSO + 10% FBS decreased to 76.2% of the unpreserved control. The cell density after cryopreservation in Bambanker hRM was 96.6%, which was significantly higher compared to the unpreserved control. The experiments were conducted three times each. Statistical significance (vs. Opti-MEM® + 10% DMSO + 10% FBS) was calculated by Dunnett's multiple comparison test (n = 3). ** p < 0.01.

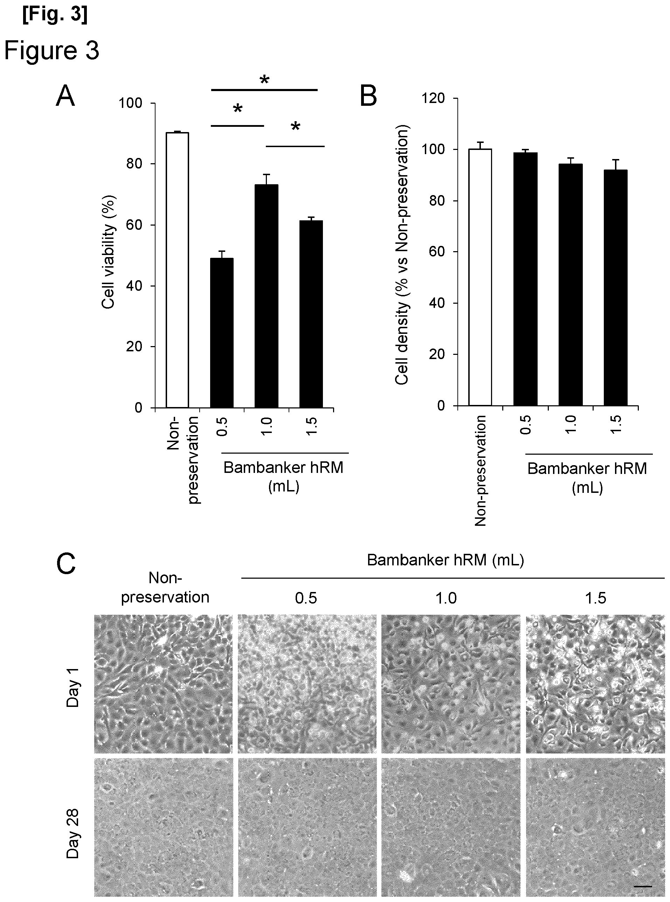

[Figure 3] Figure 3 shows optimization of the cell concentration during cryopreservation. (A) A total of 5 × 10⁵ HCECs was suspended in 0.5, 1, or 1.5 ml of Bambanker hRM and cryopreserved for 14 days. The viable HCECs collected from the frozen stock were identified by trypan blue staining. The ratio of viable HCECs was significantly higher when the cells were suspended in 1.0 ml relative to 0.5 or 1.5 ml of Bambanker hRM. The experiments were conducted three times each. Statistical significance (vs. 1.0 ml) was calculated by Dunnett's multiple comparison test (n = 3). ** p < 0.05. (B) HCECs were cultured for 28 days. The cell density was evaluated with ImageJ. The experiments were conducted three times each (n = 3). (C) In a representative day one phase contrast image, more cells that appear to be dead cells were observed in HCECs preserved in 0.5 and 1.5 ml of Bambanker hRM than in HCECs preserved in 1.0 ml of Bambanker hRM. After 28 days, HCECs preserved in 0.5, 1.0 and 1.5 ml of Bambanker hRM formed a hexagonal monolayer sheet-like structure at similar cell densities. Scale bar: 200 µm.

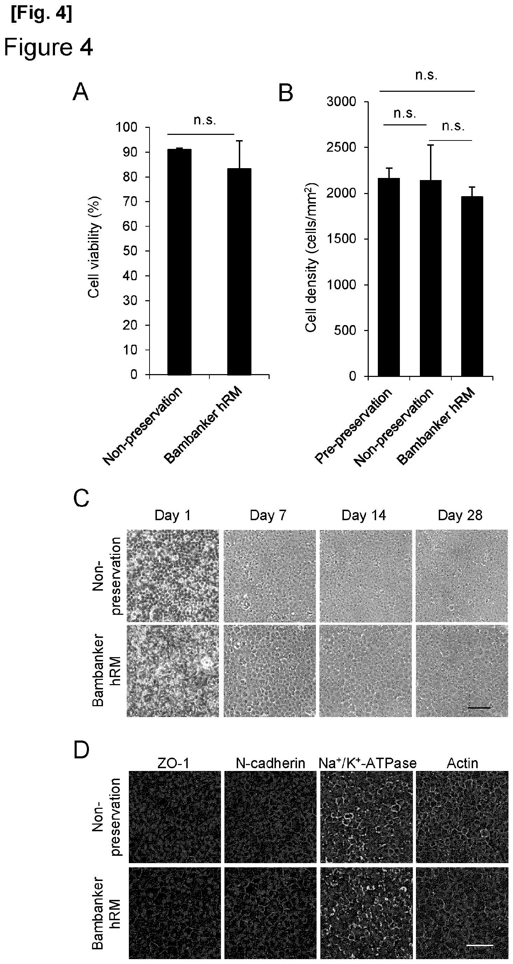

[Figure 4] Figure 4 shows the feasibility of use of Bambanker hRM in the preservation of clinical grade human corneal endothelial cells (HCECs). (A) HCECS of the same grade as clinically used cells (with a cell density > 2000 cells/mm² at passage 3 to 5) were cryopreserved using Bambanker hRM. The cell survival rate was 83.2% after 14 days of cryopreservation, and 91.0% for the unpreserved control. The experiments were conducted repeatedly. (B) HCECs were cultured for 28 days after cryopreservation. The cell density was evaluated with ImageJ. The cell density was about the same with cryopreservation relative to the cells of unpreserved control (2099 cells/mm² and 2111 cells/mm², respectively). Statistical significance (vs. unpreserved) was calculated by Student's t test (n = 4) . (C) Phase contrast microscope images show that HCECs preserved using Bambanker hRM grow in the same manner as the unpreserved control. Scale bar: 200 µm. (D) For HCECs cultured for 28 days after culture, function related markers were evaluated by immunofluorescence staining. The cell morphology was evaluated by actin staining. HCECs preserved using Bambanker hRM expressed the same markers, i.e., function related ZO-1, N-cadherin, and Na⁺/K⁺-ATPase, as those observed in the unpreserved control. Actin showed a normal corneal endothelial pattern observed in the unpreserved control in preserved HCECs . Scale bar: 200 µm.

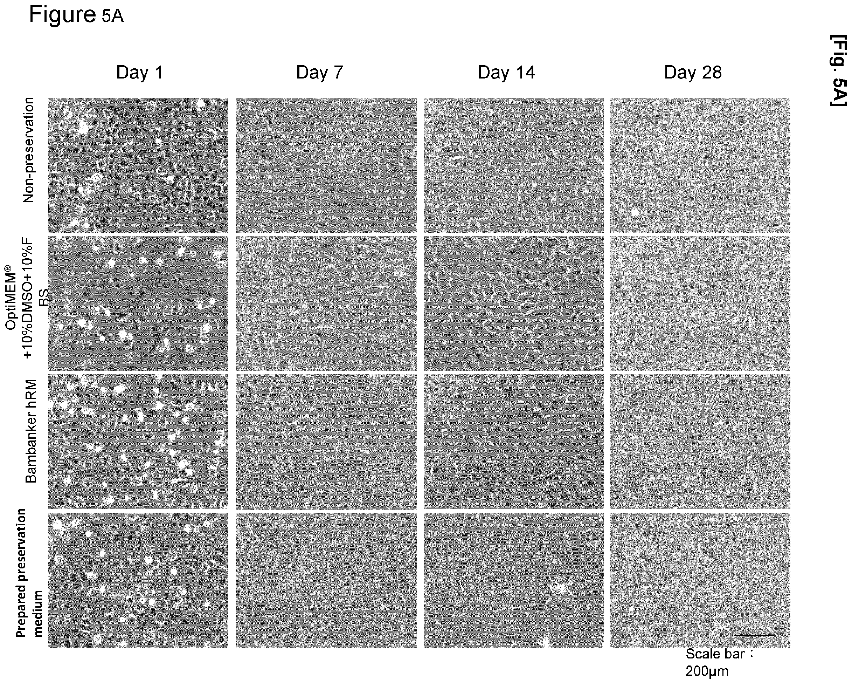

[Figure 5A] Figure **5A** shows representative examples of phase contrast microscope images of cells with cryopreservation using a cell preservation medium prepared in Example 4. For comparison, cells without cryopreservation, cells cryopreserved using Opti-MEM®-I + 10% DMSO + 10% FBS, and cells cryopreserved using Bambanker hRM were used.

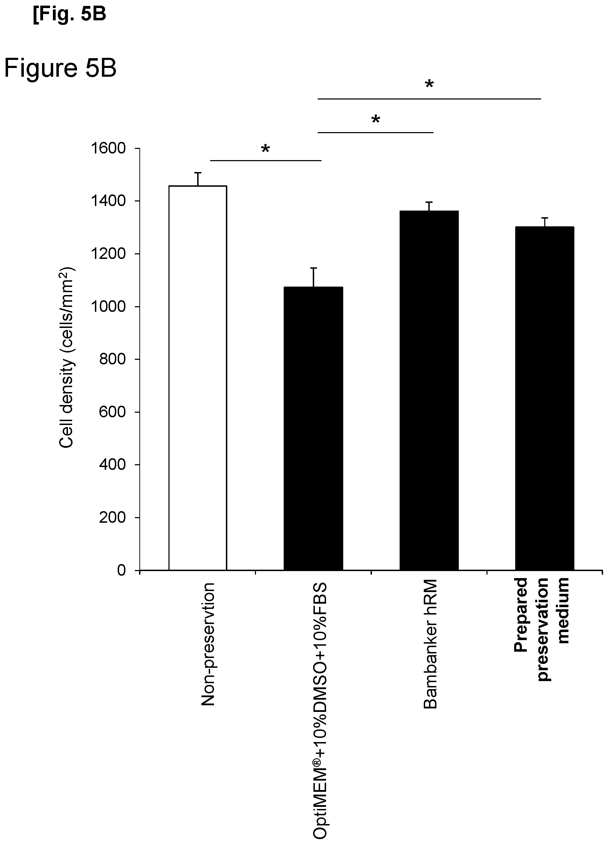

[Figure 5B] Figure **5B** shows results for cell density upon cryopreservation using the cell preservation medium prepared in Example 4. Relative to cells cryopreserved using Opti-MEM®-I + 10% DMSO + 10% FBS, the prepared cell preservation medium maintained a significantly higher cell density in the same manner as BamBanker hRM.

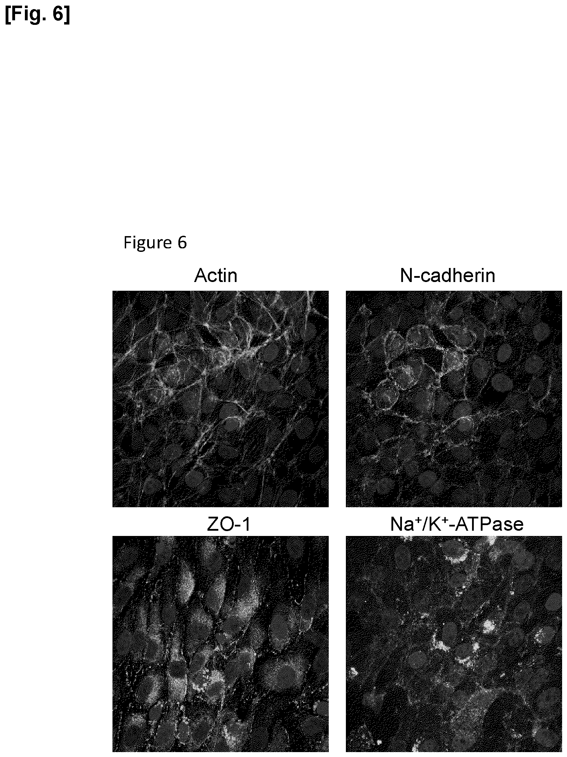

[Figure 6] Figure **6** shows results of histological examination after cultured corneal endothelium transplant using human corneal endothelial cells that were cryopreserved using the cell formulation technology of the present disclosure.

### [Description of Embodiments]

The present disclosure is described hereinafter. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present invention pertains. In case of a contradiction, the present specification (including the definitions) takes precedence. As used herein, "about" refers to a range of ± 10% of the subsequent value.

### (Preferred embodiments)

The preferred embodiments are described hereinafter. It is understood that the embodiments are exemplification of the present disclosure, so that the scope of the present disclosure is not limited to such preferred embodiments. It is understood that those skilled in the art can refer to the following preferred embodiments to readily make modifications or changes within the scope of the present disclosure. Any of these embodiments can be appropriately combined by those skilled in the art.

### (Composition)

In one aspect, the present disclosure provides a composition for preserving (e.g., cryopreserving) ocular cells or culturing the cells after preservation (e.g., cryopreservation), comprising albumin and dimethyl sulfoxide. The inventors found that the cell survival rate and/or cell density is maintained, and the cell count significantly increases in culture after preservation when ocular cells (e.g., corneal cells, typically corneal endothelial cells) are preserved using a composition comprising albumin and dimethyl sulfoxide, compared to using a conventional preservation medium. Examples of ocular cells that can be preserved with the composition of the present disclosure include corneal epithelial cells, corneal stromal cells, corneal endothelial cells, trabecular cells, retinal pigment epithelial cells, bipolar cells, ganglion cells, scleral cells, and lens epithelial cells. Such ocular cells are preferably corneal cells such as corneal epithelial cells, corneal stromal cells, and corneal endothelial cells, and most preferably corneal endothelial cells. Ocular cells can be cells derived from mammals (human, mouse, rat, hamster, rabbit, cat, dog, cow, horse, sheep, monkey, or the like), but are preferably derived from primates and particularly preferably derived from humans. It is more difficult to maintain culture of ocular cells compared to other body parts. For preservation, ocular cells often exhibit behavior that is different from other cells. Therefore, it was unknown to those skilled in the art that ocular cells, especially corneal cells, and more specifically corneal endothelial cells can be efficiently preserved. In particular, it was unknown whether ocular cells, especially corneal cells, and more specifically corneal endothelial cells can be isolated and/or preserved in a cultured state, or preserved in a transplantable state thereafter.

In some embodiments, ocular cells that can be preserved can be isolated cells or cells derived from progenitor cells (e.g., ES cells or iPS cells) . It is difficult to maintain ocular cells (particularly corneal endothelial cells) such as isolated cells or cells derived from progenitor cells compared to cells assembled as a tissue. Such cells can be preserved with a high cell survival rate and/or cell density with the composition for preservation of the present disclosure.

In some embodiments, the composition of the present disclosure is substantially free of nutritional factors for growing and/or differentiating cells during preservation.

In some embodiments, the composition of the present disclosure can be for administrating ocular cells after preservation to a subject. Ocular cells preserved using the composition of the present disclosure can be immediately administered without additional culturing, with only a simple operation such as exchanging media as needed. More specifically, the ocular cells can be administered to a subject without cell culture or incubation for 24 hours or more, 18 hours or more, 12 hours or more, preferably 6 hours or more (or any other unit of time) for the purpose of growth and/or re-differentiation of cells after exchanging at least some of the components other than cells in the composition of the present disclosure with other components such as injected medium by means such as centrifugation. Therefore, ocular cells preserved using the composition of the present disclosure can be administered to a subject without culturing. Ocular cells can also be co-administered with a ROCK inhibitor. A ROCK inhibitor can be included in a composition in advance, mixed in immediately before administration, or administered individually upon administration of cells. Examples of ROCK inhibitors include compounds disclosed in the following documents: US Patent No. 4678783, Japanese Patent No. 3421217, International Publication No. WO 95/28387, International Publication No. WO 99/20620, International Publication No. WO 99/61403, International Publication No. WO 02/076976, International Publication No. WO 02/076977, International Publication No. WO 2002/083175, International Publication No. WO 02/100833, International Publication No. WO 03/059913, International Publication No. WO 03/062227, International Publication No. WO 2004/009555, International Publication No. WO 2004/022541, International Publication No. WO 2004/108724, International Publication No. WO 2005/003101, International Publication No. WO 2005/039564, International Publication No. WO 2005/034866, International Publication No. WO 2005/037197, International Publication No. WO 2005/037198, International Publication No. WO 2005/035501, International Publication No. WO 2005/035503, International Publication No. WO 2005/035506, International Publication No. WO 2005/080394, International Publication No. WO 2005/103050, International Publication No. WO 2006/057270, and International Publication No. WO 2007/026664. Specific examples thereof include, but are not limited to, Y-27632 ((+)-trans-4-(1-aminoethyl)-1-(4-pyridylcarbamoyl)cyclohex a ne), ripasudil (4-fluoro-5-{ [(2S)-2-methyl-1,4-diazepan-1-yl]sulfonyl}isoq uinoline), fasudil (1-(5-isoquinolinesulfonyl)homopiperadine), and pharmaceutically acceptable salts thereof.

Albumin that can be used in the present disclosure can be derived from any species, such as cells derived from mammals (human, mouse, rat, hamster, rabbit, cat, dog, cow, horse, sheep, monkey, or the like), but those derived from humans are preferably used when preserving human cells. In an embodiment of preserving human cells, albumin derived from a human can be used or albumin derived from a cow can be used. The albumin used in the present disclosure is generally not used as a component of serum, but in a separated state from other components. In one example, albumin that is used is preferably purified albumin. Purification can be performed using any method in the art. The albumin concentration in a composition below about 0.001% (w/w) results in no effect, and a concentration above about 25% (w/w) results in no change in the effect. Therefore, in some embodiment, the concentration of albumin can be in the range of about 0.001% to about 25% (w/w), such as about 0.01 to about 25% (w/w), about 0.01 to about 20% (w/w), about 0.01 to about 15% (w/w), about 0.01 to about 10% (w/w), about 0.01 to % (w/w), about 5% (w/w), about 0.1 to about 25% (w/w), about 0.1 to % (w/w), about 20% (w/w), about 0.1 to about 15% (w/w), about 0.1 to about 10% (w/w), and more preferably about 0.1 to about 5% (w/w) . In a preferred embodiment, the albumin concentration can be about 1% (w/w).

The dimethyl sulfoxide used in the present disclosure can be commonly known dimethyl sulfoxide that is commercially available. The dimethyl sulfoxide concentration in a composition below about 0.1% (w/w) results in a reduced ability to preserve cells, and a concentration above about 50% (w/w) results in no change in the effect. Thus, the concentration is preferably within the range of about 0.1% (w/w) to about 50% (w/w) such as about 0.1 to about 40% (w/w), about 0.1 to about 30% (w/w), about 0.1 to about 20% (w/w), about 1 to about 50% (w/w), about 1 to about 40% (w/w), about 1 to 30% (w/w), and more preferably in the range of about 1 to about 20% (w/w) . In a preferred embodiment, the dimethyl sulfoxide concentration can be about 10% (w/w).

In some embodiments, the composition of the present disclosure can further comprise a saccharide. Although not wishing to be bound by any theory, the post-preservation viable cell rate (effective cell rate) and culture efficiency improve by further including a saccharide. Examples of saccharides include, but are not limited to, monosaccharides, disaccharides, and trisaccharides, such as glucose, galactose, fructose, ribose, trehalose, and glucose. In a preferred embodiment, a saccharide that is added is preferably a monosaccharide. In particular, glucose is preferred due to the ease of handling, but other saccharides described above can be used or any combination can be used. The saccharide concentration in a composition of less than about 0.1% (w/w) results in no effect, and a concentration above about 10% (w/w) results in no change in the effect. Thus, the concentration is in the range of about 0.1 to about 10% (w/w), such as about 0.1 to 9% (w/w), about 0.1 to 8% (w/w), about 0.1 to about 7% (w/w), about 0.1 to about 6% (w/w), about 0.1 to about 5% (w/w), about 1 to 10% (w/w), about 1 to about 9% (w/w), about 1 to about 8% (w/w), about 1 to about 7% (w/w), about 1 to about 6% (w/w), and more preferably about 1 to about 5% (w/w).

In some embodiments, the composition of the present disclosure can further comprise a phosphate ion. A phosphate ion can be used as a phosphate buffer. Dulbecco's phosphate buffer, phosphate buffer, or the like can be used.

In some embodiments, the composition of the present disclosure can comprise a metal ion. Although not wishing to be bound by any theory, the post-preservation viable cell rate (effective cell rate) after preservation and culture efficiency improve by further including a metal ion. Examples of metal ions include, but are not limited to, divalent and trivalent metal ions, such as calcium ion, magnesium ion, iron ion, zinc ion, copper ion, and aluminum ion. In a preferred embodiment, a metal ion can be a divalent metal ion. The metal ion concentration in a composition of less than about 0.0000001% (w/w) results in no effect, and a concentration above about 1% (w/w) results in no change in effect. Thus, in a specific embodiment, the metal ion concentration is in the range of about 0.0000001% to about 5% (w/w) such as about 0.1 to about 4% (w/w), about 0.1 to about 3% (w/w), about 0.1 to about 2% (w/w), about 0.1 to about 1% (w/w), about 0.01 to about 5% (w/w), about 0.01 to about 4% (w/w), about 0.01 to about 3% (w/w), about 0.01 to about 3% (w/w), about 0.01 to about 2% (w/w), about 0.01 to about 1% (w/w), and more preferably about 0.1 to about 5% (w/w) . In a specific embodiment, the composition of the present disclosure can comprise any combination of the metal ions described above. For example, the composition of the present disclosure can comprise (i) a calcium ion and magnesium or (ii) calcium, magnesium, and copper. In a more specific embodiment, the composition of the present disclosure can comprise (i) about 0.1% calcium and magnesium or (ii) 0.1 to 5% calcium, magnesium, and copper.

In a specific embodiment, the composition of the present disclosure can comprise about 0.1% (w/w) to 5% (w/w) of albumin and about 5% (w/w) to 15% (w/w) of dimethyl sulfoxide.

In a specific embodiment, the composition of the present disclosure can comprise about 1% (w/w) of albumin and about 10% (w/w) of dimethyl sulfoxide.

In a specific embodiment, the composition of the present disclosure is, for example, Bambanker®, Bambanker® hRM, or STEM-CELLBANKER®, or comprises the same. Bambanker® is preferable due to the high ability to grow cells in culture after preservation. STEM-CELLBANKER® is preferred due to the high ability to maintain the cell survival rate and cell density after preservation. Bambanker® hRM is particularly preferable due to the high ability to maintain the cell survival rate and cell density after preservation and the high ability to grow cells in culture thereafter. Bambanker® hRM comprises about 1% (w/w) of albumin, about 10% (w/w) of dimethyl sulfoxide, and other medium components. Those skilled in the art can appropriately modify the medium components. It is possible to confirm whether a medium comprising a modified medium component is within the scope of the present disclosure by using any approach that is explicitly disclosed herein or known in the art, preferably by testing the component is clinically applicable after preservation.

### (Cell formulation)

In another aspect, the present disclosure provides a cell formulation comprising corneal endothelial cells, albumin, and dimethyl sulfoxide. In some embodiment, the cell formulation of the present disclosure can be provided in a frozen state. The cell formulation of the present disclosure can comprise a therapeutically effective amount of corneal endothelial cells. Since a high cell survival rate and/or cell density is maintained even in a frozen state, a therapeutically effective amount of clinically usable normal corneal endothelial cells can be administered after thawing. Therefore, a cell formulation provided in a frozen state can be thawed immediately before administration and administered to a subject without a subsequent culturing step, such that the formulation is provided in a "ready to use" state. The cell formulation of the present disclosure can be for treating or preventing a corneal endothelial disease, disorder, or symptom of a subject. Examples of corneal endothelial disease, disorder, or symptom include, but are not limited to, Fuchs' endothelial corneal dystrophy, post-corneal transplant disorder, corneal endotheliitis, trauma, post-ophthalmic surgery disorder, post-ophthalmic laser surgery disorder, aging, posterior polymorphous dystrophy (PPD), congenital hereditary endothelial dystrophy (CHED), idiopathic corneal endothelial disorder, cytomegalovirus corneal endotheliitis, and the like.

As for constituent elements contained in a cell formulation (albumin, dimethyl sulfoxide, metal ion, saccharide, ROCK inhibitor, and the like), one or more embodiments described above (composition) can be appropriately used.

### (Preservation method)

In another aspect, the present disclosure prvoides a method for preserving ocular cells (e.g., corneal endothelial cells), comprising suspending the ocular cells in the composition described above to provide a suspension, and freezing the suspension without pre-freezing. Typically, cells are suspended in a cell preservation medium (composition of the present disclosure) in a container for low temperature preservation (tube, plate, dish, or the like) at about 1 × 10³ to about 1 × 10⁸ cells/ml, and this is preserved at about -80°C or in liquid nitrogen. If the preservation is for a short period of time, the temperature can be about -4°C to about -20°C. Ocular cells can be suspended in a cell preservation medium, dispensed into a plurality of containers for cell preservation, and cryopreserved without pre-freezing. Upon preservation, the temperature of cells suspended in the cell preservation medium can be gradually reduced with a programmable freezer or the like, or rapidly reduced directly. Regarding recovery of cryopreserved cells, cells preserved at a low temperature are melted in a thermostatic vessel at room temperature or 37°C, and the cells and the cell preservation medium are separated by an operation such as centrifugation using a centrifuge to recover the cells. The separated cells can be cultured by a common method after washing with a suitable culture medium or the like.

In a preferred embodiment, one advantageous feature of the present disclosure can be in being ready-to-use, where the cryopreserved cells can be directly administered after recovery to a patient. A method of thawing and/or recovery for direct administration in such an embodiment can be any approach, but the following is a representative approach. Specifically, cells in a frozen state can be administered by thawing according to a conventional method, and performing a simple operation such as exchanging the composition of the present disclosure with another composition by means such as centrifugation as needed without culturing cells for a certain period of time in order to grow and redifferentiate the cells, and administrating directly or by suspending the cells in a solution for administration (e.g., saline or phosphate buffered saline (PBS)) . It is understood that this is within the scope of direct administration.

### (Treatment/prevention)

In another aspect, the present disclosure provides a method for treating or preventing an ophthalmic (e.g., corneal endothelial) disease, disorder, or symptom in a subject, comprising administering a therapeutically effective amount of ocular cells (e.g., corneal endothelial cells) preserved in accordance with the aforementioned preservation method of the present disclosure to the subject.

The following procedure is provided as a representative approach.
1) preserve ocular cells (e.g., corneal endothelial cells) using the composition for preservation of the present disclosure and cryopreserve as needed;
2) thaw the ocular cells (e.g., corneal endothelial cells) preserved in 1) as needed upon treatment, and subsequently suspend the cells in an intraocular injection solution, centrifuge the suspension, and resuspend the cells into the injection solution;
3) add a ROCK inhibitor as needed; and
4) inject the composition comprising the ocular cells (e.g., corneal endothelial cells) into an eye of a subject in need thereof.

The preferred embodiments described in other parts of the present specification such as each of the section of (Composition), (Preservation method), (Cell formulation), (Cells), and (Use) can be appropriately combined and used as needed as the technologies used in treatment/prevention of the present disclosure.

### (Cells)

In another aspect, the present disclosure provides ocular cells (e.g., corneal endothelial cells) for treating or preventing an ophthalmic (e.g., corneal endothelial) disease, disorder, or symptom in a subject, wherein the ocular cells (e.g., corneal endothelial cells) are corneal endothelial cells preserved in accordance with the aforementioned preservation method of the present disclosure.

The preferred embodiments described in other parts of the present specification such as each of the section of (Composition), (Preservation method), (Cell formulation), (Treatment/prevention), and (Use) can be appropriately combined and used as needed as the technologies used in the cells of the present disclosure.

### (Use)

In another aspect, the present disclosure provides use of corneal endothelial cells in the manufacture of a drug for treating or preventing a corneal endothelial disease, disorder, or symptom in a subject, wherein the ocular cells (e.g., corneal endothelial cells) are ocular cells (e.g., corneal endothelial cells) preserved in accordance with the aforementioned preservation method of the present disclosure.

The preferred embodiments described in other parts of the present specification such as each of the section of (Composition), (Preservation method), (Cell formulation), (Treatment/prevention), and (Cells) can be appropriately combined and used as needed as the technologies used in the use of the present disclosure.

The present disclosure has been described while showing preferred embodiments to facilitate understanding. The present disclosure is described hereinafter based on the Examples. The above descriptions and the following Examples are not provided to limit the present disclosure, but are provided for the sole purpose of exemplification. Thus, the scope of the present disclosure is not limited to the embodiments and Examples that are specifically disclosed herein and is limited only by the scope of claims.

### [Examples]

The present disclosure is more specifically described hereinafter based on the Examples. It is understood that the specifically shown reagents as well as those available from Sigma-Aldrich, BASF Japan Ltd., or the like can be used as the various reagents used in the Examples.

### (Cell culture example)

The summary of exemplary cell culture in this Example is shown in Figure 1A.

Donor corneas were obtained from SightLife™. Corneas of healthy donors who were 40 years old or older were used. The corneas preserved in Optisol (Chiron Vision) at 4°C for a preservation period of 14 days or less were used in the experiments. Descemet's membranes containing the corneal endothelium were stripped from the donor corneas, and the Descemet's membranes were digested for 12 hours at 37°C with 1 mg/mL collagenase A (Roche Applied Science, 10 103 586 001) . After washing three times with OptiMEM™-I (invitrogen, 31985-088), they were seeded in one well of a 48-well plate coated with laminin 511 E8 (iMatrix-511; Nippi, 892012). The culture medium was prepared as follows. NIH-3T3 cells treated for 2 hours with 4 µg/mL of mitomycin C (Kyowa Hakkko Kirin, 4231400D1031) were seeded on a culture dish at a cell density of 1.35 × 10⁴ cells/cm². NIH-3T3 cells were cultured with a basal medium for 24 hours, and the basal medium was collected. The collected basal medium was filtered through a 0.22-µm filtration unit (Millipore, R7NA75775), and used for culturing corneal endothelial cells after adding 5 ng/mL of epithelial growth factor (invitrogen, PHG0311), 0.1 mM ascorbic acid (SIGMA-Aldrich, PHR1008), 10 µM of SB203580 (Cayman, 13067), and 1 µM of SB431542 (WAKO, 192-16541) upon use. Cultured human corneal endothelial cells at passages 5 through 10 and with a cell density of 1000 to 1500 cells/mm² were used for the screening experiments.

The following basal medium was used.

Basal medium: Opti-MEM®-I(invitrogen, 31985-088) + 8% FBS (Thermo, SH30084.03) + 5 ng/mL epithelial growth factor (invitrogen, PHG0311) + 20 µg/mL ascorbic acid (SIGMA-Aldrich, PHR1008) + 200 mg/L calcium chloride (SIGMA-Aldrich, 449709) + 0.08% chondoroitin sulfate (WAKO, 032-14613) + 50 µg/mL gentamicin (invitrogen, 15710-064).

The medium was removed from the culture dish where human corneal endothelial cells were being cultured, and 1× PBS(-) (Nippon Flour Mills Co., Ltd., 05913) preheated to 37°C was added to wash the cells. This was repeated twice. 1× PBS(-) was added again, and the cells were incubated for 3 minutes at 37°C (5% CO₂). After removing the PBS (-), TrypLE™ Select Enzyme (10X) (Thermo Fisher Scientific, A1217701) was added, and the cells were incubated for 15 minutes at 37°C (5% CO₂). The cells were then suspended in a medium. The cells were collected by centrifugation for 3 minutes at 280 G. After counting the cells, human corneal endothelial cells were centrifuged for 3 minutes at 280 G. After removing the supernatant, various cryopreservation reagents (1 ml for 5 × 10⁵ cells) were added. The human corneal endothelial cells were then frozen at -80°C by using a BICELL bio freezing vessel (Nihon Freezer Co., Ltd., BICELL) for 24 hours, and preserved in liquid nitrogen (-196°C) for 13 more days. After 14 days of cryopreservation, human corneal endothelial cells were re-cultured from the frozen stock. Briefly, cryotubes preserving human corneal endothelial cells were incubated in a 37°C water bath for 1 to 2 minutes. Human corneal endothelial cells were collected in a basal medium preheated at 37°C. The human corneal endothelial cells were then centrifuged at 190 G for 5 minutes. The supernatant was removed, and the human corneal endothelial cells were suspended in the medium. The human corneal endothelial cells were then seeded at a density of 800 cells/mm² in a culture plate. The prepared preservation medium was used as the cryopreservation medium. Opti-MEM®-I + 10% DMSO + 10% FBS was used as a control for the cryopreservation medium.

### (Example of observation with phase contrast microscope)

An exemplary method of observing cells in this Example is described.

Cryotubes preserving human corneal endothelial cells were incubated in a 37°C water bath for 1 to 2 minutes. Human corneal endothelial cells were collected in a basal medium preheated at 37 °C. The human corneal endothelial cells were then centrifuged at 190 G for 5 minutes. The supernatant was removed, and the human corneal endothelial cells were suspended in the medium. The human corneal endothelial cells were then seeded at a density of 800 cells/mm² in a well coated with laminin-511 E8 and an uncoated well after adding Y-27632 (Wako Pure Chemical Industries, Ltd., 253-00513) to the suspension. The cell morphology and cell adhesion were observed after 24 hours under a phase contrast microscope.

### (Results)

Figure 1B shows the results of cell observation. Less human corneal endothelial cells cultured after cryopreservation adhered to a plate compared to cells cultured without freezing. The inventors previously reported that laminin-511 E8 is a component of a basal membrane of a corneal endothelium, i.e., Descemet's membrane, and use of laminin-511 E8 as a cell culture substrate promotes human corneal endothelial cells. For this reason, the effect of laminin-511 E8 in cell culture after cryopreservation was evaluated. Aphase contrast image showed that the cell count increased by use of laminin-511 E8, but the number of cells adhering to a culture plate was less for cryopreserved cells compared to cells cultured without cryopreservation. Cells cultured without laminin-511 E8 exhibited a morphology of fibroblasts, but cells cultured on a plate coated with laminin-511 E8 had few fibroblasts and had a corneal endothelial cell-like morphology.

### (Example of measuring viable cells)

A typical method of measuring viable cells in this Example is described.

Cryotubes cryopreserving human corneal endothelial cells were incubated in a 37°C water bath for 1 to 2 minutes. Human corneal endothelial cells were collected in a basal medium preheated at 37 °C. The human corneal endothelial cells were then centrifuged at 190 G for 5 minutes. The supernatant was removed, and the human corneal endothelial cells were suspended in the medium. The human corneal endothelial cells were then seeded at a density of 800 cells/mm² in a well coated with laminin-511 E8 and an uncoated well after adding Y-27632 to the suspension. To find the viable cell count after 24 hours, the viable cell count was measured with Cell Titer-Glo Luminescent Cell Viability Assay by the following procedure. First, a human corneal endothelial cell suspension was prepared for calibration curves. The medium in the plate was removed. The cells were washed with Opti-MEM®-I preheated to 37°C, and then Opti-MEM®-I was added at 50 µl/well. Human corneal endothelial cells used only for calibration were seeded in an empty well at 0 cells/well, 5000 cells/well, 10000 cells/well, and 15000 cells/well. Cell Titer-Glo Luminescent Cell Viability Assay solution (Promega, G7572) was added at 50 µl/well so that medium:solution would be 1:1. The operation hereafter was performed with the light shielded. A shaker was shaken thoroughly for 2 minutes at about 120 minutes⁻¹ and incubated for 10 minutes. After incubation, 80 µl was transferred to an Assay plate (Corning, 3912, Assay plate 96 well, white polystyrene), and absorbance was measured using GloMax-Multi Detection System (Promega, E7051).

### (Results)

Figure **1C** shows the result of measuring viable cells. The cell count increased significantly due to use of laminin-511 E8. Meanwhile, the cell count after 24 hours decreased to about half for cells cryopreserved in Opti-MEM®-I + 10% DMSO + 10% FBS compared to cells without cryopreservation.

### (Example 1: Screening of cryopreservation medium)

In this Example, cryopreservation media suitable for cryopreservation of corneal endothelial cells were screened.

### (A Cell survival rate)

Cryotubes preserving human corneal endothelial cells were incubated in a 37°C water bath for 1 to 2 minutes. Human corneal endothelial cells were collected in a basal medium preheated at 37°C. The human corneal endothelial cells were then centrifuged at 190 G for 5 minutes. The supernatant was removed, and the human corneal endothelial cells were suspended in the medium. Dead cells were then stained for 10 minutes with 0.5%-Trypan Blue Stain solution (Nacalai Tesque, 29853-34). The viable cell count and the dead cell count were determined using a hemocytometer. The cell survival rate was calculated by dividing the viable cell count by the preserved cell count (5 × 10⁵ cells) in cryopreserved cells. This was calculated by dividing the viable cell count by the sum of dead cell count and viable cell count in cells without cryopreservation. CELLBANKER®2 (Nippon Seiyaku Kogyou Co., Ltd., CB031), Bambanker (Nippon Genetics Co., Ltd., CS-02-001), KM Banker (Kohjin Bio Co., Ltd., 16092005), STEM-CELLBANKER® (Nippon Seiyaku Kogyou Co., Ltd., CB047), Bambanker hRM (Nippon Genetics Co., Ltd., CS-07-001), and ReproCryo DMSO Free RM (ReproCELL, RCHEFM003) were used as the preservation medium. Opti-MEM®-I + 10% Dimethyl sulfoxide (DMSO; Nacalai Tesque, 13408-64) + 10% FBS was used as a control for the cryopreservation reagent.

### (Results)

Figure **2A** shows the results. The survival rate of cells preserved using Opti-MEM®-I + 10% DMSO + 10% FBS was 75.0%, but the ratio of viable cells was 89.2% in cells without cryopreservation. Meanwhile, use of Bambanker hRM in the preservation significantly increased the ratio of viable cells to 89.4%.

### (B Examination of ability to grow cells with Cell titer glo)

Cryotubes cryopreserving human corneal endothelial cells were incubated in a 37°C water bath for 1 to 2 minutes. Human corneal endothelial cells were collected in a basal medium preheated at 37°C. The human corneal endothelial cells were then centrifuged at 190 G for 5 minutes. The supernatant was removed, and the human corneal endothelial cells were suspended in the medium. Y-27632 was added to the cell suspension, and the cells were seeded on a 96-well plate coated with laminin-511 E8. To find the ability to grow cells after 1 day, 7 days, 14 days, and 28 days, the viable cell count was measured with Cell Titer-Glo Luminescent Cell Viability Assay by the following procedure. First, a human corneal endothelial cell suspension was prepared for a calibration curve. The medium in the plate was removed. The cells were washed with Opti-MEM®-I preheated to 37°C, and then Opti-MEM®-I was added at 50 µl/well. Human corneal endothelial cells for the calibration curve were seeded in empty wells . Cell Titer-Glo Luminescent Cell Viability Assay solution (Promega, G7572) was added at 50 µl/well so that medium:solution would be 1:1. The operation hereafter was performed with the light shielded. A shaker was shaken thoroughly for 2 minutes at about 120 minutes⁻¹ and incubated for 10 minutes. After incubation, 80 µl was transferred to an Assay plate (Corning, 3912, Assay plate 96 well, white polystyrene), and absorbance was measured using GloMax-Multi Detection System (Promega, E7051).

### (Results)

Figure **2B** shows the results. All of the cryopreserved cells were cultured over 28 days. Bambanker hRM and Bambanker particularly tended to allow cells to grow faster than other preserved cells.

### (C Cell density)

Cryopreserved cryotubes were incubated in a 37°C water bath for 1 to 2 minutes. Human corneal endothelial cells were collected in a basal medium preheated at 37 °C. The human corneal endothelial cells were then centrifuged at 190 G for 5 minutes. The supernatant was removed, and the human corneal endothelial cells were suspended in the medium. Y-27632 was then added to the cell suspension, and the cells were seeded at a density of 800 cells/mm² on a culture plate coated with laminin-511 E8. After washing the cells that have reached confluence on day 28 after seeding twice with 1× PBS(-), the cells were incubated for 3 minutes at 37°C (5% CO₂). After washing the cells that have reached confluence on day 28 after seeding twice with 1× PBS (-), the cells were incubated for 3 minutes at 37°C (5% CO₂). A picture was then taken using a phase contrast microscope at a 200× magnification. The cell density was measured by using a measurement software, ImageJ software (National Institutes of Health) . First, the taken phase contrast microscope picture was uploaded into the software, and the cells were counted using the counting function. Analysis was performed when 100 cells were counted to measure the cell density. Dunnett's multiple comparison test was used for testing. The results are represented as mean ± standard deviation.

### (Results)

Figure **2C** shows the results. If it is assumed that the density of cells that have not been cryopreserved is 100, the density of cryopreserved cells using Opti-MEM®-I + 10% DMSO + 10% FBS decreased to 76.2%. However, the density of cells that were cryopreserved using Bambanker hRM maintained the cell density at 96.6%.

### (Example 2: Study of quantity of preservation medium)

This Example studied the suitable amount of cryopreservation medium used in cryopreservation.

### (A Cell survival rate)

The medium was removed from the culture dish where human corneal endothelial cells were being cultured, and 1× PBS(-) (Nippon Flour Mills Co., Ltd., 05913) preheated to 37°C was added to wash the cells. This was repeated twice. 1× PBS(-) was added again, and the cells were incubated for 3 minutes at 37°C (5% CO₂). After removing the PBS (-), TrypLE™ Select Enzyme (10X) (Thermo Fisher Scientific, A1217701) was added, and the cells were incubated for 15 minutes at 37°C (5% CO₂). The cells were then suspended in a medium. The cells were collected by centrifugation for 3 minutes at 280 G. After counting the cells, the suspension was dispensed in each 15 ml centrifuge tube and centrifuged for 3 minutes at 280 G. After removing the supernatant, 0.5 ml, 1 ml, and 1.5 ml of cryopreservation reagents were added so that each would have a concentration of 5 × 10⁵ cells per 1 ml. The human corneal endothelial cells were then frozen at -80°C by using a BICELL bio freezing vessel (Nihon Freezer Co., Ltd., BICELL) for 24 hours, and preserved in liquid nitrogen (-196°C) for 13 more days. After 14 days of cryopreservation, human corneal endothelial cells were re-cultured from the frozen stock. Briefly, cryotubes preserving human corneal endothelial cells were incubated in a 37°C water bath for 1 to 2 minutes. Human corneal endothelial cells were collected in a basal medium preheated at 37°C. The human corneal endothelial cells were then centrifuged at 190 G for 5 minutes. The supernatant was removed, and the human corneal endothelial cells were suspended in the medium. Dead cells were then stained for 10 minutes with 0.5%-Trypan Blue Stain solution (Nacalai Tesque, 29853-34). The viable cell count and the dead cell count were determined using a hemocytometer. The cell survival rate was calculated by dividing the viable cell count by the preserved cell count (5 × 10⁵ cells) in cryopreserved cells. This was calculated by dividing the viable cell count by the sum of dead cell count and viable cell count in cells without cryopreservation. Bambanker hRM was used as the preservation medium. Dunnett's multiple comparison test was used for testing. The results are represented as mean ± standard deviation.

### (Results)

Figure **3A** shows the results. The cell survival rate was significantly higher when the cells were suspended in 1.0 ml of Bambanker hRM than when suspended in 0.5 or 1.5 ml of Bambanker hRM.

### (B Cell density)

Cryopreserved cryotubes were incubated in a 37°C water bath for 1 to 2 minutes. Human corneal endothelial cells were collected in a basal medium preheated at 37°C. The human corneal endothelial cells were then centrifuged at 190 G for 5 minutes. The supernatant was removed, and the human corneal endothelial cells were suspended in the medium. Y-27632 was then added to the cell suspension, and the cells were seeded at a density of 800 cells/mm² on a culture plate coated with laminin-511 E8. After washing the cells that have reached confluence on day 28 after seeding twice with 1× PBS(-), the cells were incubated for 3 minutes at 37°C (5% CO₂). A picture was then taken using a phase contrast microscope at a 200× magnification. The cell density was measured by using a measurement software, ImageJ software (National Institutes of Health). First, the taken phase contrast microscope picture was uploaded into the software, and the cells were counted using the counting function. Analysis was performed when 100 cells were counted to measure the cell density. Dunnett's multiple comparison test was used for testing. The results are represented as mean ± standard deviation.

### (Results)

When cells were suspended in 0.5, 1.0, and 1.5 ml of Bambanker hRM, the cell densities were all at the same level.

### (C Phase contrast microscope image)

Cryotubes cryopreserving human corneal endothelial cells were incubated in a 37°C water bath for 1 to 2 minutes. Human corneal endothelial cells were collected in a basal medium preheated at 37°C. The human corneal endothelial cells were then centrifuged at 190 G for 5 minutes. The supernatant was removed, and the human corneal endothelial cells were suspended in the medium. Y-27632 was then added to the cell suspension, and the cells were seeded at a density of 800 cells/mm² in a culture plate coated with laminin-511 E8. The cell morphology and cell adhesion were observed after 1 day, 7 days, 14 days, and 28 days under a phase contrast microscope.

### (Results)

Figure **3C** shows the results. The results show that on day 1 after seeding, cells preserved with 0.5 ml and 1.5 ml of Bambanker hRM had a higher number of dead cells and a lower number of cells adhering to a culture plate compared to the cells preserved with 1.0 ml of Bambanker hRM. On day 28, cells preserved with 0.5, 1.0, and 1.5 ml of Bambanker hRM formed a hexagonal and a monolayer sheet like structures at the same cell density.

### (Example 3: Evaluation of cryopreservation in high density cells)

### (A Cell survival rate)

To evaluation the feasibility of a cryopreservation protocol, cells of clinically usable quality with a cell density of 2000 cells/mm² or greater and at passage 3 to 5 were used as the clinically used cells to study the effect of Bambanker hRM on cell preservation. The medium was removed from the culture dish where human corneal endothelial cells were being cultured, and 1× PBS(-) (Nippon Flour Mills Co., Ltd., 05913) preheated to 37°C was added to wash the cells. This was repeated twice. 1× PBS(-) was added again, and the cells were incubated for 3 minutes at 37°C (5% CO₂). After removing the PBS(-), TrypLE™ Select Enzyme (10X) (Thermo Fisher Scientific, A1217701) was added, and the cells were incubated for 15 minutes at 37°C (5% CO₂). The cells were then suspended in a medium. The cells were collected by centrifugation for 3 minutes at 280 G. After counting the cells, human corneal endothelial cells were centrifuged for 3 minutes at 280 G. After removing the supernatant, cryopreservation reagents were added (1 ml for 5 × 10⁵ cells). The human corneal endothelial cells were then frozen at -80°C by using a BICELL bio freezing vessel (Nihon Freezer Co., Ltd., BICELL) for 24 hours, and preserved in liquid nitrogen (-196°C) for 13 more days. After 14 days of cryopreservation, human corneal endothelial cells were re-cultured from the frozen stock. Briefly, cryotubes preserving human corneal endothelial cells were incubated in a 37 °C water bath for 1 to 2 minutes. Human corneal endothelial cells were collected in a basal medium preheated at 37°C. The human corneal endothelial cells were then centrifuged at 190 G for 5 minutes. The supernatant was removed, and the human corneal endothelial cells were suspended in the medium. Dead cells were then stained for 10 minutes with 0.5%-Trypan Blue Stain solution (Nacalai Tesque, 29853-34). The viable cell count and the dead cell count were determined using a hemocytometer. The cell survival rate was calculated by dividing the viable cell count by the preserved cell count (5 × 10⁵ cells) in cryopreserved cells. This was calculated by dividing the viable cell count by the sum of dead cell count and viable cell count in cells without cryopreservation. The statistical significance (p value) of the mean value in a two-sample comparison was determined by Student's t test. The results are represented as mean ± standard deviation. Bambanker hRM was used as the preservation medium.

### (Results)

The cell survival rate after cryopreservation was 83.2%. A significant decrease was not found in comparison to the 91.0% cell survival rate of cells without cryoperservation.

### (B Cell density)

Cryopreserved cryotubes were incubated in a 37°C water bath for 1 to 2 minutes. Human corneal endothelial cells were collected in a basal medium preheated at 37°C. The human corneal endothelial cells were then centrifuged at 190 G for 5 minutes. The supernatant was removed, and the human corneal endothelial cells were suspended in the medium. Y-27632 was then added to the cell suspension, and the cells were seeded at a density of 2500 cells/mm² on a culture plate coated with laminin-511 E8. After washing the cells that have reached confluence on day 28 after seeding twice with 1× PBS(-), the cells were incubated for 3 minutes at 37°C (5% CO₂). A picture was then taken using a phase contrast microscope at a 200× magnification. The cell density was measured by using a measurement software, ImageJ software (National Institutes of Health). First, the taken phase contrast microscope picture was uploaded into the software, and the cells were counted using the counting function. Analysis was performed when 100 cells were counted to measure the cell density. Dunnett's multiple comparison test was used for testing. The results are represented as mean ± standard deviation.

### (Results)

Figure **4B** shows the results. The density of cryopreserved cells was 1959 cells/mm², reaching a density similar to the density of 2138 cells/mm² of the cells without cryoperservation.

### (C Phase contrast microscope)

Cryotubes cryopreserving human corneal endothelial cells were incubated in a 37°C water bath for 1 to 2 minutes. Human corneal endothelial cells were collected in a basal medium preheated at 37°C. The human corneal endothelial cells were then centrifuged at 190 G for 5 minutes. The supernatant was removed, and the human corneal endothelial cells were suspended in the medium. Y-27632 was then added to the cell suspension, and the cells were seeded at a density of 2500 cells/mm² on a culture plate coated with laminin-511 E8. The cell morphology and cell adhesion were observed after 1 day, 7 days, 14 days, and 28 days under a phase contrast microscope.

### (Results)

Figure **4C** shows the results. The cells preserved using Bambanker hRM grew in the same manner as cells that were not cryopreserved and formed hexagonal and monolayer sheet like structures.

### (D Immunostaining)

Cells cultured on a 48-well plate were washed three times with 1× PBS (+) and fixed in 0.5% paraformaldehyde (PFA: WAKO, 163-20145) for 45 minutes. After removing the PFA, the cells were washed three times with 1× PBS(-). The cells were then permeabilized with 1% Triton X-100 (Nacalai Tesque, 287229-25) for 5 minutes. After removing the 1% Triton X-100, the cells were washed three times with 1× PBS (-) . The cells were incubated with 2% bovine serum albumin (BSA: SIGMA-Aldrich, A9418-5G) for 30 minutes at 37°C for blocking. The cells were shaken with primary antibodies for 45 minutes at 37°C at 25 min⁻¹. ZO-1 (1: 200, invitrogen, 617300), N-cadherin (1: 300, BD Biosciences, 610921), and Na⁺/K⁺⁻ATPase (1:200, Merck Millipore, 05-369) were used as the primary antibodies. After removing the primary antibodies, the cells were washed three times with 1× PBS(-) and shaken for 5 minutes each at 25 min-¹. Either Alexa Fluor® 488-conjugated goat anti-rabbit IgG (Thermo Fisher Scientific, A21206) or Alexa Fluor® 594-conjugated goat anti-mouse IgG (Thermo Fisher Scientific, A-11005) was diluted at 1: 500 in BSA, and the samples were shaken with the secondary antibodies for 45 minutes at 37°C at 25 minutes⁻¹. Actin in samples was shaken with a 1:200 diluted Alexa Fluor® 546-conjugated Phalloidin (invitrogen, A22283), and nuclei in samples were shaken with 1:1000 diluted 4',6-diamidino-2-phenylindole dihydrochloride (DAPI, Dojindo Laboratories, D523) for 45 minutes at 37°C at 25 minutes⁻¹. After removing the secondary antibodies, the cells were washed three times with 1× PBS (-) and shaken for 5 minutes each at 25 min⁻¹. After removing the 1× PBS(-), an antifading agent was added, and the samples were examined with a fluorescence microscope (Keyence, BZ-9000).

### (Results)

Figure **4D** shows the results. A marker of tight junction ZO-1, marker of adherence junction N-cadherin, and marker of pump function Na⁺/K⁺⁻ATPase were confirmed to be expressed on the cell membrane of cells cryopreserved using Bambanker hRM, in the same manner as cells without cryopreservation. Actin staining indicated that actin distributed at cell cortex without fibroblastic change expresses a normal corneal endothelial pattern in both cryopreserved cells and cells without cryopreservation.

In view of the above, the composition of the present disclosure was found to be capable of preservation without compromising the cell survival rate or cell density even at a cell density at a clinically applicable level (e.g., 2000 cells/mm² or greater) of cell density.

### (Example 4: Study of efficacy of the prepared preservation medium)

0.36 g of sodium bicarbonate (Nacalai Tesque, 31213-15), 60 g of D- (+)-Glucose ≥ 99.5% (GC) (SIGMA-ALDRICH, G7021-100G), 0.72 g of HEPES (Nacalai Tesque, 17514-02), and 19.29 g of PBS (-) powder (Nippon Flour Mills Co., Ltd., 05913) were weighted using an analytical balance (SHIMADZU, AUW220D), and dissolved in ultrapure water to prepare a 500 ml solution. This is used as reagent (1).

Likewise, 0.5 g of calcium chloride (Nacalai Tesque, 06729-55) and 0.5 g of magnesium chloride hexahydrate (Nacalai Tesque, 20909-55) were each weighted using an analytical balance, and dissolved in ultrapure water to prepare a 500 ml solution. This is used as reagent (2).

Reagent (1) and reagent (2) were sterilized for 20 minutes at 121°C with an autoclave. 12.5 ml of reagent (1) and 27.5 ml of sterilized water (Nacalai Tesque, 06442-95) were measured out and admixed. 5 ml of DMSO (Nacalai Tesque, 13408-64) was then added and admixed. Furthermore, 5 ml of reagent (2) was added and admixed. 0.5 g of bovine serum albumin (SIGMA-ALDRICH, A9418-5G) weighted with an analytical balance was added and admixed. Finally, the cell preservation medium was adjusted by filtration with Millex-GV, 0.22 µm and PVDF, 33 mm (Millipore, R7NA75775). The following Table 1 shows the reagents used.

**Table 1**

| Name of reagent | Manufacturer | Model No. |
|---|---|---|
| Sodium bicarbonate | Nacalai Tesque | 31213-15 |
| µD-(+)-Glucose | SIGMA-ALDRICH | G7021-100G |
| HEPES | Nacalai Tesque | 17514-02 |
| PBS(-) powder | Nippon Flour Mills Co., Ltd. | 05913 |
| Calcium chloride | Nacalai Tesque | 06729-55 |
| Magnesium chloride hexahydrate | Nacalai Tesque | 20909-55 |
| Sterilized water | Nacalai Tesque | 06442-95 |
| DMSO | Nacalai Tesque | 13408-64 |
| Bovine Serum Albumins | SIGMA-ALDRICH | A9418-5G |

### (A Observation with phase contrast microscope)

The medium was removed from the culture dish where human corneal endothelial cells were being cultured, and 1× PBS(-) (Nippon Flour Mills Co., Ltd., 05913) preheated to 37°C was added to wash the cells. This was repeated twice. 1× PBS(-) was added again, and the cells were incubated for 3 minutes at 37°C (5% CO₂). After removing the PBS (-), TrypLE™ Select Enzyme (10X) (Thermo Fisher Scientific, A1217701) was added, and the cells were incubated for 15 minutes at 37°C (5% CO₂). The cells were then suspended in a medium. The cells were collected by centrifugation for 3 minutes at 280 G. After counting the cells, human corneal endothelial cells were centrifuged for 3 minutes at 280 G. After removing the supernatant, cryopreservation reagents (1 ml for 5 × 10⁵ cells) were added. The human corneal endothelial cells were then frozen at -80°C by using a BICELL bio freezing vessel (Nihon Freezer Co., Ltd., BICELL) for 24 hours, and preserved in liquid nitrogen (-196°C) for 13 more days. Cryotubes cryopreserving human corneal endothelial cells were incubated in a 37°C water bath for 1 to 2 minutes. Human corneal endothelial cells were collected in a basal medium preheated at 37°C. The human corneal endothelial cells were then centrifuged at 190 G for 5 minutes. The supernatant was removed, and the human corneal endothelial cells were suspended in the medium. Y-27632 was then added to the cell suspension, and the cells were seeded at a density of 800 cells/mm² on a culture plate coated with laminin-511 E8. The cell morphology and cell adhesion were observed after 1 day, 7 days, 14 days, and 28 days under a phase contrast microscope.

### (Results)

Figure **5A** shows the results . On day 1 after culture, cells cultured after cryopreservation using Opti-MEM®-I + 10% DMSO + 10% FBS had lower number of cells adhering to a plate compared to cells cultured without cryopreservation. Further, cells cryopreserved using the prepared preservation medium were shown to have more adherent cells in a plate compared to cells cryopreserved using Opti-MEM®-I + 10% DMSO + 10% FBS. It was shown that cells cryopreserved using Bambanker hRM or prepared preservation medium had the same density as cells cultured without cryopreservation on day 28 after culture. On the other hand, the density of cells cryopreserved using Opti-MEM®-I+ 10% DMSO + 10% FBS decreased.

### (B Cell density)

Cryopreserved cryotubes were incubated in a 37°C water bath for 1 to 2 minutes. Human corneal endothelial cells were collected in a basal medium preheated at 37°C. The human corneal endothelial cells were then centrifuged at 190 G for 5 minutes. The supernatant was removed, and the human corneal endothelial cells were suspended in the medium. Y-27632 was then added to the cell suspension, and the cells were seeded at a density of 800 cells/mm² on a culture plate coated with laminin-511 E8. After washing the cells that have reached confluence on day 28 after seeding twice with 1× PBS(-), the cells were incubated for 3 minutes at 37°C (5% CO₂). A picture was then taken using a phase contrast microscope at a 200× magnification. The cell density was measured by using a measurement software, ImageJ software (National Institutes of Health). First, the taken phase contrast microscope picture was uploaded into the software, and the cells were counted using the counting function. Analysis was performed when 100 cells were counted to measure the cell density. The test used the Tukey-Kramer multiple comparison test. The results are represented as mean ± standard deviation.

### (Results)

Figure **5B** shows the results . The density of cells cultured without cryopreservation was 1457 cells/mm², whereas the densities of cells cryopreserved using Bambanker hRM (1% albumin and 10% dimethyl sulfoxide) and the prepared preservation solution were 1362 cells/mm² and 1301 cells/mm², respectively, indicating that similar densities are maintained. Meanwhile, the density of cells cryopreserved using Opti-MEM®-I + 10% DMSO + 10% FBS significantly decreased to 1073 cells/mm².

### (Example 5: Corneal endothelial cell transplant)

In this Example, corneal endothelial cells preserved in accordance with the method of the present disclosure were transplanted into rabbit bullous keratopathy model.

### (Materials and Methods)

### (Culture)

Human corneal endothelial cells obtained from donor corneas provided by SightLife™ were used in the following experiment. All corneas were placed in Optisol (Chiron Vision) before use in the experiment and preserved at 4°C for less than 14 days. Descemet's membranes containing the corneal endothelium were stripped from the donor corneas, and the Descemet's membranes were digested for 12 hours at 37°C with 1 mg/mL collagenase A (Roche Applied Science, 10 103 586 001) . After washing three times with OptiMEM™-I (invitrogen, 31985-088), they were seeded in one well of a 48-well plate coated with laminin 511 E8 (iMatrix-511; Nippi, 892012). The culture medium was prepared as follows. Briefly, NIH-3T3 cells treated for 2 hours with 4 µg/mL of mitomycin C (Kyowa Hakkko Kirin, 4231400D1031) were seeded on a culture dish at a cell density of 2 × 10⁴ cells/cm². NIH-3T3 cells were cultured with a basal medium for 24 hours, and the basal medium was collected. The collected basal medium was filtered through a 0.22 µm filtration unit (Millipore, R7NA75775), and used for culturing human corneal endothelial cells after adding 5 ng/mL of epithelial growth factor (invitrogen, PHG0311), 0.1mM ascorbic acid (SIGMA-Aldrich, PHR1008), 10 µM of SB203580 (Cayman, 13067), and 1 µM of SB431542 (WAKO, 192-16541) upon use.

Basal medium: Opti-MEM®-I(invitrogen, 31985-088) + 8% FBS (Thermo, SH30084.03) + 5 ng/mL epithelial growth factor (invitrogen, PHG0311) + 20 µg/mL ascorbic acid (SIGMA-Aldrich, PHR1008) + 200 mg/L calcium chloride (SIGMA-Aldrich, 449709) + 0.08% chondoroitin sulfate (WAKO, 032-14613) + 50 µg/mL gentamicin (invitrogen, 15710-064).

### (Cryopreservation of human corneal endothelial cells)

The medium was removed from the culture dish where human corneal endothelial cells were being cultured, and 1× PBS(-) (Nippon Flour Mills Co., Ltd., 05913) preheated to 37°C was added to wash the cells. This was repeated twice. 1× PBS(-) was added again, and the cells were incubated for 3 minutes at 37°C (5% CO₂). After removing the PBS (-), TrypLE™ Select Enzyme (10X) (Thermo Fisher Scientific, A1217701) was added, and the cells were incubated for 15 minutes at 37°C (5% CO₂). The cells were then suspended in a medium. The cells were collected by centrifugation for 3 minutes at 280 G. After counting the cells, human corneal endothelial cells were centrifuged for 3 minutes at 280 G. After removing the supernatant, Bambanker hRM (Nippon Genetics Co., Ltd., CS-07-001) (1 ml for 5 × 10⁵ cells) were added. The human corneal endothelial cells were then frozen at -80°C by using a BICELL bio freezing vessel (Nihon Freezer Co., Ltd., BICELL) for 24 hours, and preserved in liquid nitrogen (-196°C) for 5 days. The cells were cryopreserved for 6 days.

### (Preparation of cells for transplantation)

Human corneal endothelial cells were cryopreserved for 6 days using Bambanker hRM. Cryopreserved cryotubes were incubated in a 37 °C water bath for 1 to 2 minutes. Human corneal endothelial cells were collected by pipetting twice with a basal medium preheated at 37°C. The human corneal endothelial cells were then centrifuged at 190 G for 5 minutes. The supernatant was removed, and the human corneal endothelial cells were resuspended in Opti-MEM®-I at a concentration of 5.0 × 10⁵ cells/300 µl. Y-27632 (Wako Pure Chemical Industries, Ltd., 253-00513) was added so that the final concentration would be 100 µM.

### (Transplantation method)

Cryopreserved and cultured corneal endothelial cells were used for cultured corneal endothelial transplant using a rabbit bullous keratopathy model. A corneal endothelium of a rabbit was mechanically stripped with a 20-gauge silicone needle (Soft Tapered Needle; Inami & Co., Ltd., Tokyo, Japan) to create a bullous keratopathy model. 5.0 × 10⁵ cultured human corneal endothelial cells initiated from a frozen state from the post-cryopreservation cultured human corneal endothelial cells created above, which were suspended in 300 µl of Opti-MEM®-I with an addition of Y-27632 resulting in the final concentration of 100 µM, were injected into the anterior chamber of a bullous keratopathy model. The model was laid face down for three hours.

### (Histological test)

Corneal tissue extracted from a rabbit was fixed in 4% paraformaldehyde (PFA: WAKO, 163-20145) for 10 minutes at room temperature (RT). The cells were then permeabilized with 1% Triton X-100 (Nacalai Tesque, 287229-25) for 5 minutes and blocked with 2% bovine serum albumin (BSA: SIGMA-Aldrich, A9418-5G) for 1 hour. To study the phenotype of the regenerated corneal endothelial tissue, ZO-1 and N-cadherin were used as markers related to the barrier function of cells, and Na⁺/K⁺⁻ATPase was used as a marker related to the pump function as the primary antibodies. For ZO-1, N-cadherin, and Na⁺/K⁺⁻ATPase staining, ZO-1 (1:300, Thermo Fisher Scientific, 33-9100), N-cadherin (1:300, BD Biosciences, 610921), and Na⁺/K⁺⁻ATPase (1:300, Merck Millipore, 05-369) were used, respectively. As the secondary antibodies, Alexa Fluor® 594-conjugated goat anti-mouse IgG (Thermo Fisher Scientific, A-11005) was diluted to 1:1000 in BSA and used.

For actin, a 1:400 diluted Alexa Fluor® 488-conjugated Phalloidin (invitrogen, A12379) was used. For nuclei, 1:1000 diluted 4',6-diamidino-2-phenylindole dihydrochloride (DAPI: Dojindo Laboratories, D523) was used. The samples were then examined with a confocal microscope (Leica Microsystems, DM14000 B).

### (Results)

Figure **6** shows the results of histological study after cultured corneal endothelial transplant using cryopreserved human corneal endothelial cells. As shown in Figure **6****,** a gene product expressed in normal corneal endothelial cells was expressed. More specifically, Na⁺/K⁺⁻ATPase indicating the pump function and ZO-1 (barrier function) indicating tight junction were expressed. The figure also shows that N-cadherin indicating adherence junction is also expressed normally. Phalloidin staining also indicates that cells have actin morphology of a polygonal monolayer, which is the same as normal cells. The above results revealed that corneal endothelial tissue with a normal function can be regnerated in the body by injecting human corneal endothelial cells cultured after cryopreservation. The results show that Bambanker hRM can provide human corneal endothelial cells in a so-called "ready to use" state, under which human corneal endothelial cells can be cryopreserved and used immediately in transplant without undergoing a process such as culturing cells initiated from a frozen state.

### (Example 6: Preparation of cell formulation of corneal endothelial cells)

This Example provides a cell formulation of corneal endothelial cells provided based on the present disclosure.

### (Materials and Methods)

Human corneal endothelial cells are cryopreserved for 30 days using Bambanker hRM. Cryopreserved cryotubes are incubated in a 37 °C water bath for 1 to 2 minutes . Human corneal endothelial cells are collected by pipetting twice with a basal medium (e.g., Opti-MEM®-I or the like) preheated at 37°C. The human corneal endothelial cells are then centrifuged at 190 G for 5 minutes. The supernatant is removed, and the human corneal endothelial cells are resuspended in Opti-MEM®-I at a concentration of 5.0 × 10⁵ cells/300 µl. Y-27632 (Wako Pure Chemical Industries, Ltd., 253-00513) is added so that the final concentration would be 100 µM.

### (Use)

The cell formulation prepared in this Example can be used directly, and clinically applied after exchanging a solution with phosphate buffered saline.

### (Example 7: Transplant therapy using cell formulation of corneal endothelial cells)

This Example performs transplant therapy using a cell formulation of corneal endothelial cells of the present disclosure.

This Example uses the technology of Examples 1-5 or the cell formulation prepared as in Example 6.

A corneal endothelium of a bullous keratopathy patient is mechanically stripped with a 20-gauge silicone needle (Soft Tapered Needle; Inami & Co., Ltd., Tokyo, Japan) in the range of about an 8 mm diameter. The cryopreserved cultured human corneal endothelial cells prepared in Example 6 are injected in the anterior chamber using a 26 G needle, and laid face down for three hours.

The effect of the cultured human corneal endothelial cells is studied using any indicator that is known in the art. For example, the effect can be confirmed using an approach such as measurement of the cell density or vision examination.

As disclosed above, the present disclosure is exemplified by the use of its preferred embodiments. However, it is understood that the scope of the present disclosure should be interpreted based solely on the Claims. It is also understood that any patent, any patent application, and any references cited herein should be incorporated herein by reference in the same manner as the contents are specifically described herein.

The present application claims priority to Japanese Patent Application No. 2018-163238 filed on August 31, 2018. The entire content thereof is incorporated herein by reference.

### [Industrial Applicability]

A composition for preserving corneal endothelial cells, or culturing the cells after preservation, is provided. The composition of the present disclosure can preserve ocular cells at a high cell survival rate and/or cell density. Since ocular cells preserved in such a manner can be used in cell transplant or the like, ocular cells can be utilized in the field of drug development or the like.

## Claims

1. A composition for use in preserving ocular cells, or culturing the cells after preservation, comprising albumin and dimethyl sulfoxide.

2. The composition of claim 1, wherein the ocular cells comprise corneal cells.

3. The composition of claim 1, wherein the ocular cells comprise corneal endothelial cells.

4. The composition of any one of claims 1 to 3, wherein the ocular cells are isolated cells or cells derived from progenitor cells.

5. The composition of any one of claims 1 to 4, wherein the albumin is derived from a cow.

6. The composition of any one of claims 1 to 5, further comprising a saccharide.

7. The composition of claim 6, wherein the saccharide is a monosaccharide.

8. The composition of claim 6, wherein the saccharide is a glucose.

9. The composition of any one of claims 1 to 8, further comprising a phosphate ion.

10. The composition of any one of claims 1 to 9, further comprising a metal ion.

11. The composition of claim 10, wherein the metal ion is a divalent metal ion.

12. The composition of claim 10, wherein the metal ion is selected from the group consisting of a calcium ion, a magnesium ion, an iron ion, a zinc ion, a copper ion, an aluminum ion, and any combination thereof.

13. The composition of claim 10, wherein the composition comprises (i) a calcium ion and magnesium or (ii) calcium, magnesium, and copper.

14. The composition of any one of claims 1 to 13, wherein a concentration of the albumin is within the range of about 0.01 to about 25% (w/w).

15. The composition of any one of claims 1 to 13, wherein a concentration of the albumin is within the range of about 0.1 to about 5% (w/w).

16. The composition of any one of claims 1 to 13, wherein a concentration of the albumin is about 1% (w/w).

17. The composition of any one of claims 1 to 16, wherein a concentration of the dimethyl sulfoxide is within the range of about 0.1 to about 50% (w/w).

18. The composition of any one of claims 1 to 16, wherein a concentration of the dimethyl sulfoxide is within the range of about 1 to about 20% (w/w).

19. The composition of any one of claims 1 to 16, wherein a concentration of the dimethyl sulfoxide is about 10% (w/w).

20. The composition of any one of claims 10 to 19, wherein a concentration of the metal ion is within the range of about 0.0000001 to 5% (w/w).

21. The composition of any one of claims 10 to 19, wherein a concentration of the metal ion is within the range of about 0.1 to about 5% (w/w).

22. The composition of any one of claims 10 to 19, wherein the composition comprises (i) about 0.1% calcium and magnesium or (ii) about 0.1 to about 5% calcium, magnesium, and copper.

23. The composition of any one of claims 6 to 22, wherein a concentration of the saccharide is within the range of about 0.1 to about 10% (w/w).

24. The composition of any one of claims 6 to 22, wherein a concentration of the saccharide is within the range of about 1 to about 5% (w/w).

25. The composition of any one of claims 1 to 24, wherein the preservation is cryopreservation.

26. The composition of any one of claims 1 to 25, wherein the composition is Bambanker®.

27. The composition of any one of claims 1 to 26, wherein the composition is for administering corneal endothelial cells after preservation to a subject.

28. The composition of claim 27, wherein the corneal endothelial cells after preservation are **characterized by** being administered to a subject without culturing.

29. The composition of claim 27 or 28, wherein the corneal endothelial cells are **characterized by** being co-administered with a ROCK inhibitor.

30. The composition of claim 29, wherein the ROCK inhibitor is selected from Y-27632, ripasudil, fasudil, or a pharmaceutically acceptable salt thereof.

31. A method for preserving ocular cells, comprising:
suspending ocular cells in the composition of any one of claims 1 to 30 to provide a suspension; and
freezing the suspension.

32. The method of claim 31, wherein the ocular cells are **characterized by** being suspended in the composition at about 1 × 10³ to about 1 × 10⁸ cells/ml.

33. The method of claim 31 or 32, wherein the freezing the suspension comprises freezing without pre-freezing.

34. The method of claim 33, wherein the freezing without pre-freezing comprises freezing rapidly at about -80°C.

35. A frozen cell product comprising corneal endothelial cells that have been cryopreserved in accordance with the method of any one of claims 31 to 34.

36. A cell formulation comprising corneal endothelial cells, albumin, and dimethyl sulfoxide.

37. The cell formulation of claim 36, provided in a frozen state.

38. The cell formulation of claim 36 or 37 for treating or preventing a corneal endothelial disease, disorder, or symptom.

39. The cell formulation of any one of claims 36 to 38, wherein the albumin is derived from a cow.

40. The cell formulation of any one of claims 36 to 39, further comprising a saccharide.

41. The cell formulation of claim 40, wherein the saccharide is a monosaccharide.

42. The cell formulation of claim 41, wherein the saccharide is a glucose.

43. The cell formulation of any one of claims 36 to 42, further comprising a phosphate ion.

44. The cell formulation of any one of claims 36 to 43, further comprising a metal ion.

45. The cell formulation of claim 44, wherein the metal ion is a divalent metal ion.

46. The cell formulation of claim 44, wherein the metal ion is selected from the group consisting of a calcium ion, a magnesium ion, an iron ion, a zinc ion, a copper ion, an aluminum ion, and any combination thereof.

47. The cell formulation of claim 44, wherein the cell formulation comprises (i) a calcium ion and a magnesium ion or (ii) a calcium ion, a magnesium ion, and a copper ion.

48. The cell formulation of any one of claims 36 to 47, wherein a concentration of the albumin is within the range of about 0.01 to about 25% (w/w).

49. The cell formulation of any one of claims 36 to 47, wherein a concentration of the albumin is within the range of about 0.1 to about 5% (w/w).

50. The cell formulation of any one of claims 36 to 48, wherein a concentration of the dimethyl sulfoxide is within the range of about 0.1 to about 50% (w/w).

51. The cell formulation of any one of claims 36 to 48, wherein a concentration of the dimethyl sulfoxide is within the range of about 1 to about 20% (w/w).

52. The cell formulation of any one of claims 44 to 51, wherein a concentration of the metal ion is within the range of about 0.0000001 to about 5% (w/w).

53. The cell formulation of any one of claims 44 to 51, wherein a concentration of the metal ion is within the range of about 0.1 to about 5% (w/w).

54. The cell formulation of any one of claims 44 to 51, wherein the cell formulation comprises (i) about 0.1% calcium ion and magnesium ion or (ii) about 0.1 to about 5% calcium ion, magnesium ion, and copper ion.

55. The cell formulation of any one of claims 40 to 54, wherein a concentration of the saccharide is within the range of about 0.1 to about 10% (w/w).

56. The cell formulation of any one of claims 40 to 54, wherein a concentration of the saccharide is within the range of about 1 to about 5% (w/w).

57. The cell formulation of any one of claims 36 to 56, wherein the corneal endothelial cells are at a concentration of about 1 × 10³ to about 1 × 10⁸ cells/ml.

58. The cell formulation of any one of claims 36 to 57, further comprising a ROCK inhibitor.

59. The cell formulation of claim 58, wherein the ROCK inhibitor is selected from Y-27632, ripasudil, fasudil, or a pharmaceutically acceptable salt thereof.

60. A method for treating or preventing a corneal endothelial disease, disorder, or symptom in a subject, comprising administering to the subject a therapeutically effective amount of corneal endothelial cells preserved in accordance with the method of any one of claims 31 to 34.

61. The method of claim 60, wherein the corneal endothelial cells are administered in conjunction with a ROCK inhibitor.

62. The method of claim 60 or 61, wherein the ROCK inhibitor is selected from Y-27632, ripasudil, fasudil, or a pharmaceutically acceptable salt thereof.

63. The method of any one of claims 56 to 58 without a step of culturing corneal endothelial cells.

64. Corneal endothelial cells for treating or preventing a corneal endothelial disease, disorder, or symptom in a subject, wherein the corneal endothelial cells are corneal endothelial cells preserved in accordance with the method of any one of claims 31 to 34.

65. The corneal endothelial cells of claim 64, wherein the corneal endothelial cells are **characterized by** being administered in conjunction with a ROCK inhibitor.

66. The corneal endothelial cells of claim 64 or 65, wherein the ROCK inhibitor is selected from Y-27632, ripasudil, fasudil, or a pharmaceutically acceptable salt thereof.

67. The method of any one of claims 64 to 66, wherein corneal endothelial cells after preservation are **characterized by** being administered without further culturing.
[Claim] Use of corneal endothelial cells in the manufacture of a drug for treating or preventing corneal endothelial disease, disorder, or symptom in a subject, wherein the corneal endothelial cells are corneal endothelial cells preserved in accordance with the method of any one of claims 31 to 34.

68. The use of claim 68, wherein the corneal endothelial cells are **characterized by** being administered in conjunction with a ROCK inhibitor.

69. The use of claim 68 or 69, wherein the ROCK inhibitor is selected from Y-27632, ripasudil, fasudil, or a pharmaceutically acceptable salt thereof.

70. The use of any one of claims 68 to 70, wherein corneal endothelial cells after preservation are **characterized by** being administered without further culturing.
